# EUROPÄISCHE PATENTANMELDUNG

(11) **EP 3 141 270 A1**
(43) Veröffentlichungstag der Anmeldung: **15.03.2017**
(21) Anmeldenummer: 15184856.1
(22) Anmeldetag: 11.09.2015
(51) Int. Cl.: A61M 1/10

(54) **BLUTPUMPE, VORZUGSWEISE ZUR UNTERSTÜTZUNG EINES HERZENS**

(71) Anmelder: Berlin Heart GmbH, 12247 Berlin (DE)
(72) Erfinder: CHOUB, Leonid, 10585 Berlin (DE); GRAICHEN, Kurt, 13189 Berlin (DE); MÜLLER, Jörg, 10439 Berlin (DE); NÜSSER, Peter, 14532 Kleinmachnow (DE); WISNIEWSKI, Adrian, 10963 Berlin (DE)
(74) Vertreter: Pfenning, Meinig & Partner mbB

(57) **Zusammenfassung**

Die Anmeldung betrifft eine Blutpumpe, welche ein Gehäuse mit einem stromauf gelegenen Einlass (4), einem stromab gelegenen Auslass (6) und einen drehbar gelagerten Rotor (20) mit einer Achse (15) und einer Beschaufelung (28) umfasst. Der Rotor umfasst ferner eine Zylinderhülse oder einen Zylinder (24), wobei die Beschaufelung an einer Außenfläche des Zylinders angeordnet ist. Der Rotor ist in einer axialen Richtung magnetisch gelagert und umfasst mindestens einen an der Beschaufelung befestigten, die Beschaufelung radial außen umlaufend und in axialer Richtung magnetisierten ersten Ring (32) und ferner einen, den ersten Ring außen umlaufenden zweiten magnetisierten Ringabschnitt (14) zur Ausbildung eines axialen Magnetlagers.

## Beschreibung

Gegenstand der vorliegenden Anmeldung ist eine Blutpumpe gemäß dem Oberbegriff des Anspruchs 1.

Blutpumpen, beispielsweise zur Unterstützung eines Herzens, werden bei Patienten mit Herz- oder Gefäßschwächen eingesetzt. Die in dieser Anmeldung beschriebenen Blutpumpen können beispielsweise als Ventricular Assist Devices (VADs) zur Unterstützung des linken Ventrikels, des rechten Ventrikels oder in einem System mit zwei Pumpen für beide Ventrikel eingesetzt werden.

Im Stand der Technik sind verschiedene Blutpumpen bekannt. Beispielsweise beschreibt die US 2014/0322020 ein VAD mit einer Radialpumpe. Radialpumpen weisen einen Rotor mit einer Beschaufelung auf, wobei sich die Beschaufelung von einem Pumpeneinlass zu einem Pumpenauslass hin radial aufweitet.

Axialpumpen sind im Stand der Technik hinreichend bekannt. So zeigt beispielsweise die Druckschrift US 8 668 473 B2 eine Pumpe mit einem hydrodynamisch gelagerten Rotor, wobei in die Beschaufelung des Rotors gegebenenfalls Permanentmagnete eingelassen sind. Aus der Kombination von hydrodynamischem und magnetischem Lager soll eine axiale Lagerung der Axialpumpe möglich sein.

Aufgabe des Gegenstandes der vorliegenden Anmeldung ist es, eine Blutpumpe zur Verfügung zu stellen, welche einfach aufgebaut ist.

Erfindungsgemäß wird die Aufgabe durch eine Blutpumpe beispielsweise gemäß dem Anspruch 1 gelöst.

In einer Ausführungsform umfasst die Blutpumpe ein Gehäuse mit einem stromauf gelegenen Einlass, einem stromab gelegenen Auslass und einem zwischen dem Einlass und dem Auslass drehbar gelagerten Rotor mit einer Drehachse und einer Beschaufelung, Der Rotor ist in axialer Richtung magnetisch gelagert.

Ferner umfasst der Rotor eine Zylinderhülse oder einen Zylinder bzw. Nabe, wobei die Beschaufelung an einer Außenfläche der Zylinderhülse bzw. des Zylinders angeordnet ist. Eine Zylinderhülse unterscheidet sich von einem Zylinder dadurch, dass diese von der Achse aus gesehen eine zylinderförmige oder kegelstumpfförmige Ausnehmung besitzt. In einigen Varianten kann der Rotor von seinem stromauf gelegenen Ende bis zu seinem stromab gelegenen Ende die Form eines Zylinders haben. in anderen Ausführungsbeispielen kann der Zylinder bzw. die Zylinderhülse vom stromauf gelegenen Ende der Beschaufelung bis zum stromab gelegenen Ende der Beschaufelung reichen. In einigen Ausführungsformen kann der Rotor zu seinem stromab gelegenen Ende hin konisch zulaufen.

Ferner umfasst der Rotor einen, an der Beschaufelung befestigten, die Beschaufelung radial außen umlaufenden und in axialer Richtung magnetisierten ersten Ring. Korrespondierend hierzu ist ein den ersten Ring außen umlaufender, zweiter magnetisierter Ringabschnitt vorhanden, welcher in einem ersten axialen Gehäuseabschnitt des Gehäuses untergebracht ist. Der erste magnetisierte Ring, welcher an der Beschaufelung befestigt ist, bildet im Zusammenspiel mit dem zweiten magnetisierten Ringabschnitt ein magnetisches Axiallager.

Die Verwendung eines ersten Rings, welcher die Beschaufelung radial außen umläuft, im Zusammenspiel mit dem korrespondierend dazu ausgerichteten zweiten Ringabschnitt, bietet aufgrund der Symmetrie des Rings und des geringen radialen Abstands zwischen dem ersten Ring und dem zweiten Ringabschnitt eine besonders gute Kopplung und somit eine besonders einfach herzustellende axiale Lagerung. In einigen Ausführungsbeispielen umfasst die axiale Lagerung des Rotors im Gehäuse neben der magnetischen Lagerung keine weiteren Lager, wie beispielsweise ein hydrodynamisches Lager. In anderen Varianten kann neben dem magnetischen Axiallager auch ein weiteres, beispielsweise hydrodynamisches Lager vorhanden sein.

In einigen Ausführungsformen kann der erste Ring bzw. der zweite Ringabschnitt beispielsweise aus Weicheisen oder Permanentmagneten bestehen oder diese umfassen. Dabei ist in einigen Ausführungsformen zumindest eines der beiden Elemente des ersten Rings und des zweiten Ringabschnitts aus einem Permanentmagneten aufgebaut, um eine bessere Kopplung zwischen dem ersten Ring und dem zweiten magnetisierten Ringabschnitt zu bewirken.

In einigen Ausführungsformen ist der erste Ring am stromauf gelegenen Ende des Rotors angeordnet. Dabei kann der Ring in axialer Richtung bündig bzw. nahezu bündig mit dem stromauf gelegenen Beginn des zylindrischen Abschnitts des Rotors sein. In einer weiteren Ausführungsform kann der zweite magnetisierte Ringabschnitt ebenfalls als geschlossener Ring ausgebildet sein. In diesem Fall ist eine Anordnung des ersten und zweiten Rings zueinander besonders einfach herzustellen. Die axiale Magnetisierung des ersten Rings soll von der Magnetisierungsrichtung des weiteren Rings um 180° abweichen. Hierdurch wird eine passive axiale Magnetlagerung gebildet.

In einer weiteren Ausführungsform ist die Länge des ersten Rings in axialer Richtung kleiner als die Länge des zweiten Ringabschnitts in axialer Richtung.

In einer weiteren Ausführungsform ist der erste Gehäuseabschnitt, in welchem der stromauf gelegene Teil des Rotors angeordnet ist, im Wesentlichen als Zylinderhülse ausgebildet. Auf diese Weise lässt sich insbesondere in den Ausführungsformen, in welchen kein weiteres Vor- oder Nachleitrad vorhanden ist, die Pumpe auf besonders einfache Art und Weise montieren. Der Rotor kann im Wesentlichen einfach in den Einlass "eingeführt" werden und wird in der gewünschten Position fixiert.

In einer weiteren Ausführungsform umfasst die Blutpumpe einen weiteren Rotorabschnitt, welcher mit einem dritten Ringabschnitt, vorzugsweise einem dritten Ring versehen ist. Korrespondierend zum dritten Ringabschnitt ist im zweiten axialen Gehäuseabschnitt ein vierter, zum dritten Ringabschnitt korrespondierender, Ringabschnitt im Gehäuse angeordnet. Mit dem Vorhandensein eines weiteren Ringpaares kann die Wirkung des axialen Lagers verstärkt werden. In einer weiteren Ausführungsform sind der erste und der dritte Ringabschnitt axial voneinander beabstandet. Dabei können der erste Ringabschnitt an einem stromauf gelegenen Ende der Beschaufelung und der dritte Ringabschnitt an einem stromab gelegenen Ende der Beschaufelung angeordnet sein.

In einer weiteren Ausführungsform ist die Beschaufelung in Form mindestens einer Wendel ausgebildet. Unter einer Wendel wird vorliegend eine helixartig um die Rotoraußenfläche verlaufende Beschaufelung verstanden. Dabei ist es in einigen Ausführungsformen vorteilhaft, wenn die Beschaufelung zwei oder mehr Wendeln umfasst, welche in Umfangsrichtung betrachtet in regelmäßigen Abständen voneinander auf der Außenfläche der Zylinderhülse bzw. des Zylinders angeordnet sind.

Ferner ist in einigen Ausführungsbeispielen vorgesehen, dass jeweils eine Wendel sich mindestens mit einem Umschlingungswinkel von 360° entlang des Rotors erstreckt.

In einer weiteren Ausführungsform ist die Dicke der Beschaufelung geringer als die sich zwischen zwei Wendeln erstreckende Breite des Strömungskanals, durch welchen das Fluid transportiert wird. Der Abstand zwischen zwei Wendeln wird dabei quer zur Achse des Rotors gemessen. Dieser Abstand ist dabei breiter als die Dicke einer der Wendeln.

In einer weiteren Ausführungsform ist der Rotor in axialer Richtung ausschließlich durch das axiale Magnetlager gelagert. Dabei ist unter dem Begriff "ausschließlich" zu verstehen, dass motorbedingte Lagereffekte ebenfalls unter dem axialen Magnetlager inbegriffen sind. Dabei kann das axiale Magnetlager als passives axiales Magnetlager, d.h. ohne aktive Steuerung der Position oder als aktives axiales Magnetlager ausgebildet sein. Bei einem aktiven axialen Magnetlager ist beispielsweise stromauf und/oder stromab des zweiten Ringabschnitts im Gehäuse eine Steuerspule angeordnet, mit welcher die auf den magnetisierten ersten Ring einwirkende Magnetkraft einstellbar ist und so die Position des Rotors ebenfalls einstellbar ist.

In einer weiteren Ausführungsform ist das axiale Magnetlager derart ausgebildet, dass ein stromab gelegenes Ende der Beschaufelung vollständig stromauf des Strömungsauslasses gelagert ist. Diese Ausführungsform ist sowohl bei reinen Axialpumpen, welche das Fluid in einem koaxial zum Einlass angeordneten Auslass befördern, möglich, als auch bei einer sogenannten Tangentialpumpe, deren Auslass um einen Winkel von mehr als 45°, vorzugsweise zwischen 80° und 100°, gegenüber dem Einlass verdreht ist. Als Auslass wird hierbei derjenige Teil der Pumpe betrachtet, welcher sich an den zylindrischen Abschnitt, in welchem der Rotor der Blutpumpe gelagert ist, anschließt, sofern das Blut lediglich durch die zusätzliche Radialkomponente in den Auslass gedrückt werden kann. In einer Ausführungsform schließt sich an den Rotor ein Spiralauslass an, der insbesondere die Umfangskomponente der Geschwindigkeit aufnimmt und durch Verzögerung der Geschwindigkeit statischen Druck rückgewinnt.

In einer weiteren Ausführungsform ist die Beschaufelung derart auf dem Rotor angeordnet, dass ein stromab gelegenes Ende des Rotors stromab eines stromab gelegenen Endes der Beschaufelung liegt. In anderen Worten erstreckt sich der Rotor stromab weiter als die Beschaufelung, d.h. ein stromab gelegener Abschnitt des Rotors ist beschaufelungsfrei. Dies kann unter anderem den Vorteil haben, dass die Strömungsverhältnisse stabilisiert werden können.

In einer weiteren Ausführungsform ist ein radialer Abstand zwischen einer Innenfläche des Gehäuses und einer Außenfläche des ersten Ringabschnitts derart, dass zwischen der Innenfläche des Gehäuses und der Außenfläche des ersten Ringabschnitts ein hydrodynamisches Lager ausgebildet ist. Dieses hydrodynamische Lager wirkt in radialer Richtung und stabilisiert den Rotor radial. Unter einem Lager wird hierbei verstanden, dass ein hydrodynamisches Lager die alleinige radiale Lagerung übernehmen kann. In anderen Varianten kann das hydrodynamische Lager derart ausgebildet sein, dass dieses zusätzlich zu einem weiteren, beispielsweise magnetischen radialen Lager vorhanden ist.

In einer weiteren Ausführungsform ist ein radialer Abstand zwischen einer Innenfläche des Gehäuses und einer Außenfläche des ersten Rings derart gewählt, dass zwischen der Innenfläche des Gehäuses und der Außenfläche des ersten Rings kein hydrodynamisches Lager ausgebildet ist. Dies kann beispielsweise dann der Fall sein, wenn der Abstand zwischen besagter Außenfläche und Innenfläche groß ist. Dennoch kann der Ring in diesem Fall als radiales Dämpfungsglied wirken, indem auf den Rotor wirkende Stöße, welche zu einer Schwingung des Rotors führen, durch den Ring hydrodynamisch abgedämpft und der Rotor in seiner Bewegung stabilisiert wird. Ob das Lager nun alleinig tragend oder lediglich ein Dämpfungsglied ist, hängt somit vom Abstand zwischen der Innenfläche des Gehäuses und der Außenfläche des ersten Rings und der axialen Abmessung des Ringpaares ab.

In einigen Ausführungsbeispielen ist zwischen dem Einlass und dem Auslass ein Dorn angeordnet, welcher sich vorzugsweise vom Auslass aufwärts erstreckt. Ferner weist der Rotor eine Zylinderhülse auf und ist auf dem Dorn drehbar gelagert. Beispiele einer derartigen Blutpumpe können beispielsweise in der taggleichen Anmeldung unter dem internen Aktenzeichen 157EP 0367 gefunden werden. Der Dorn ist dabei oftmals als sich zylinderförmig in das Gehäuse erstreckender Dorn ausgebildet. An seinem stromauf gelegenen Ende kann der Dorn abgerundet sein, um einen geringeren Strömungswiderstand für das zu fördernde Fluid zu bilden. Bei den Ausführungsformen, welche einen Dorn vorsehen, auf welchem der Rotor drehbar gelagert ist, kann in einigen Varianten zwischen einer Mantelfläche des Dorns und einer Innenfläche des Rotors ein hydrodynamisches Lager ausgebildet sein. Dieses hydrodynamische Lager übernimmt vorzugsweise die radiale Lagerung des Rotors innerhalb der Blutpumpe. In den Ausführungsformen, welche einen Dorn vorsehen, kann ein den Rotor antreibender Stator im Dorn angeordnet sein. Ferner kann sowohl in den Ausführungsbeispielen mit oder ohne Dorn der Stator im ersten Gehäuseabschnitt den Rotor radial außen umlaufend angeordnet sein. Ferner ist in einigen Ausführungsbeispielen vorgesehen, dass der Stator proximal, d.h. stromab des Rotors angeordnet ist.

In einer weiteren Ausführungsform sind der Dorn und das Gehäuse bzw. der durch das Gehäuse definierte Innenraum zumindest abschnittsweise koaxial zueinander ausgerichtet.

Weitere Ausführungsbeispiele und Varianten werden anhand der nachfolgenden Figuren und Figurenbeschreibung erläutert. Es zeigen:
- Fig. 1: Längsschnitt durch eine Ausführungsform einer Pumpe;
- Fig. 2: weitere Ausführungsform einer Pumpe im Längsschnitt;
- Fig. 3: weitere Ausführungsform einer Pumpe im Längsschnitt;
- Fig. 4: Querschnitt durch einen Rotor, wie er in den Pumpen der Fign. 1 oder 2 eingesetzt werden kann;
- Fig. 5: weitere Ausführungsform eines Rotors für die Pumpen;
- Fig. 6: Längsschnitt durch eine Pumpe mit einem auf einem Dorn gelagerten Rotor;
- Fig. 7: Querschnitt durch die Pumpe der Fig. 6.

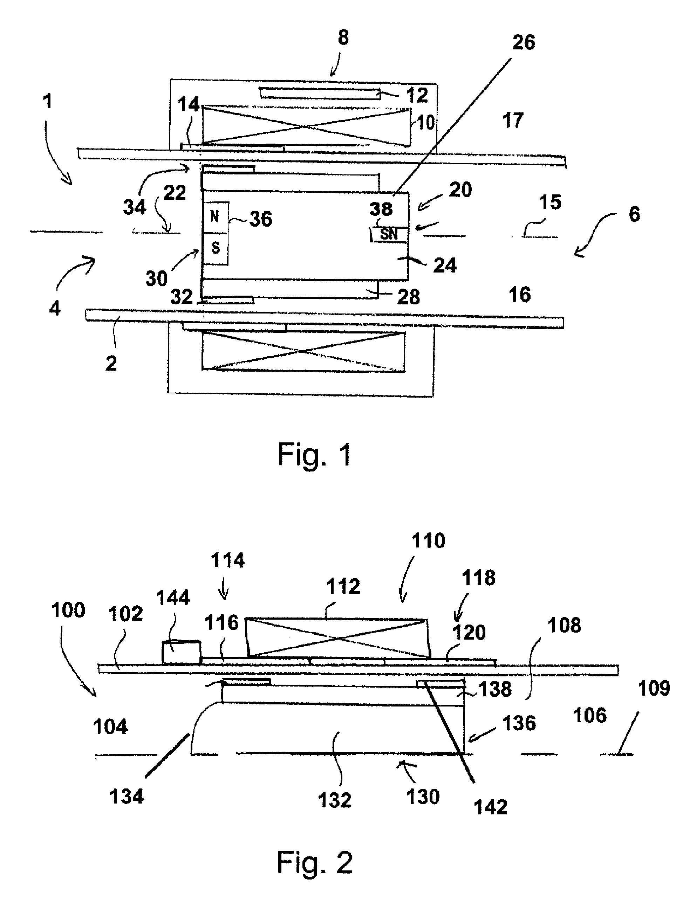

In der Fig. 1 ist ein Längsschnitt durch eine Ausführungsform einer Pumpe dargestellt. Die Blutpumpe 1 umfasst ein Gehäuse 2, welches einen stromauf gelegenen Einlass 4 und einen stromab gelegenen Auslass 6 aufweist. In einem ersten axialen Gehäuseabschnitt 8 befindet sich ein Stator 10, welcher konfiguriert ist, einen nachfolgend näher erläuterten Rotor in Drehung zu versetzen. Ferner umfasst der erste axiale Gehäuseabschnitt 8 einen Pumpencontroller 12, welcher zur Steuerung beispielsweise des Stators ausgebildet ist. Über den Pumpencontroller 12 kann die dargestellte Blutpumpe 1 mittels eines Kabels mit weiteren Komponenten, wie beispielsweise einer Batterie, einem Ladegerät oder einer Steuereinheit verbunden werden. Im ersten axialen Gehäuseabschnitt 8 befindet sich zudem ein magnetisierter Ringabschnitt 14, welcher in axialer Richtung magnetisiert ist, Dabei befindet sich der magnetische Nordpol stromauf, der magnetische Südpol stromab. Das Gehäuse 2 ist rotationssymmetrisch um die Achse 15 aufgebaut. Dies betrifft sowohl den als Ring ausgebildeten magnetisierten Ringabschnitt 14 als auch den Stator 10. Der Pumpencontroller 12 überspannt lediglich einen Abschnitt innerhalb des axialen Gehäuseabschnitts 8. In einem Innenraum 16 des Gehäuses 2 ist ein Rotor 20 angeordnet, dessen Achse 22 koaxial zur Achse 15 des Gehäuses 2 verläuft. Der Rotor 20 umfasst einen Zylinder 24, an dessen Außenfläche 26 eine Beschaufelung 28 angeordnet ist, Die Beschaufelung 28 erstreckt sich vom stromauf gelegenen Ende 30 des Rotors 20 stromab, wobei die Beschaufelung jedoch stromauf des stromab gelegenen Endes des Rotors endet. Die Beschaufelung kann jedoch auch stromabwärts des Beginns des Rotors beginnen und sich stromabwärts entlang der Nabe erstrecken, Die Beschaufelung 28 ist im vorliegenden Beispiel durch mehrere, beispielsweise drei, Wendeln gebildet. Am stromauf gelegenen Ende 30 des Rotors bzw. des in diesem Bereich angeordneten Abschnitts der Beschaufelung 28 ist ein erster Ring 32 angeordnet. Dieser Ring ist beispielsweise aus Weicheisen gefertigt. Im Zusammenspiel mit dem magnetisierten Ringabschnitt 14 bildet der Ring 32 ein axiales Magnetlager 34. Der Ring 32 könnte alternativ auch als Permanentmagnet ausgebildet sein. Dabei ist es in einer Ausführungsform vorgesehen, die Magnetisierung entgegen der Magnetisierungsrichtung des magnetisierten Ringabschnitts 14 zu wählen. Innerhalb des Zylinders 24 des Rotors 20 sind weitere Komponenten angeordnet. So können im Innern des Zylinders 24 beispielsweise Permanentmagnete 36 angeordnet sein, welche mittels des Stators 10 den Rotor in Rotation versetzen können. Diese Permanentmagnete 36 sind dabei, quer zur Achse 22 betrachtet, in Nordsüd- bzw. Südnordrichtung ausgerichtet. Des Weiteren können innerhalb des Zylinders 24 weitere Permanentmagnete 38 angeordnet sein, welche im Wesentlichen als Teil eines Sensorsystems eingesetzt werden können. So können die weiteren Permanentmagnete 38 beispielsweise dafür verwendet werden, im Zusammenwirken mit einer nicht in der Fig. 1 eingezeichneten Sensorspule Positionsänderungen des Rotors in axialer Richtung zu erfassen und/oder mittels mindestens einer nicht dargestellten Steuerspule zu bewirken und an den Pumpencontroller weiterzuleiten.

Im vorliegenden Ausführungsbeispiel sind sowohl der Innenraum 16 des Gehäuses 2 als auch der Rotor 20 im Wesentlichen zylinderförmig ausgebildet. Verjüngungen des Innenraums 16 sind aufgrund der gewählten passiven axialen Lagerung nicht notwendig.

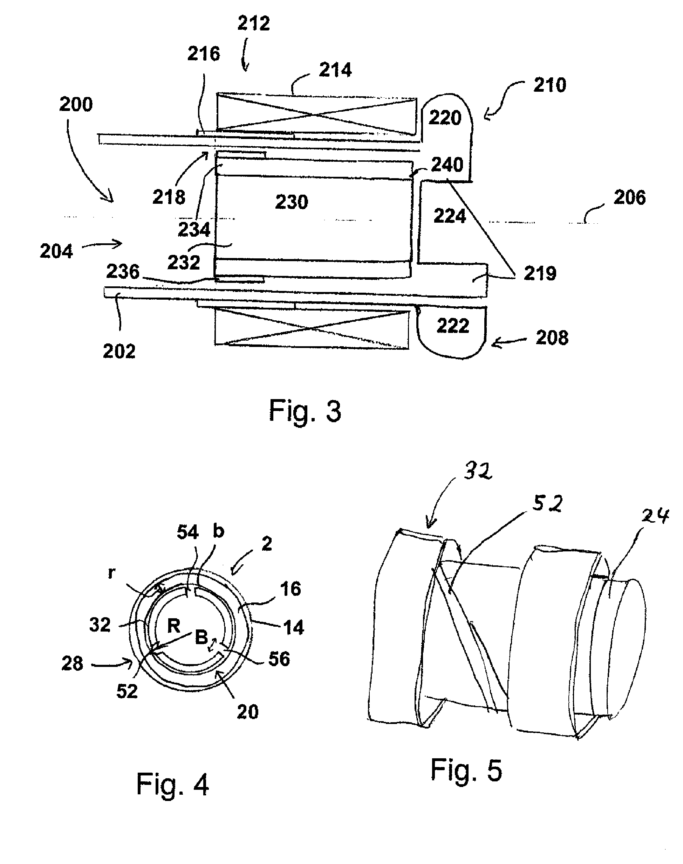

Der Rotor 20 kann beispielsweise aus Titan oder anderen biokompatiblen Materialien gefertigt sein. Als Material für das Gehäuse bietet sich ebenfalls Titan oder ein anderer biokompatibler Werkstoff an. Der axiale Gehäuseabschnitt 8 ist gegenüber dem Außenraum 17 der Pumpe fluidisch dicht. Obgleich in der Fig. 1 eine Axialpumpe mit koaxial zueinander ausgerichtetem Einlass und Auslass dargestellt ist, kann der Rotor, der Stator als auch das axiale Magnetlager in Verbindung mit einem Einlass und einem zum Einlass verdrehten Auslass, wie er beispielsweise in der Fig. 5 später beschrieben wird, verwendet werden.

In der Fig. 2 ist eine zu der Fig. 1 alternative Anordnung des Rotors innerhalb des Innenraums des Gehäuses 2 dargestellt. Die Blutpumpe 100 umfasst ein Gehäuse 102 mit einem Einlass 104 und einem Auslass 106. Der Einlass 104 und der Auslass 106 sind koaxial zueinander ausgerichtet. Der Innenraum 108 des Gehäuses 102 ist rotationssymmetrisch als Zylinder ausgebildet und lediglich von der Achse 109 sich radial nach außen erstreckend dargestellt. In einem Gehäuseabschnitt 110 ist ein Stator 112 angeordnet, welcher im Wesentlichen die Funktionen des Stators 10 der Fig. 1 erfüllt. Ferner umfasst der Gehäuseabschnitt 110 in seinem stromauf gelegenen Abschnitt 114 einen magnetisierten Ring 116 und in seinem stromab gelegenen Abschnitt 118 einen weiteren Ring 120, dessen Magnetisierung der Magnetisierung des Rings 116 entgegengesetzt ist. Der Rotor 130 umfasst einen zylinderförmigen Abschnitt 132 und ein stromauf bzw. ein stromab gelegenes Ende 134 bzw. 136, welche im Wesentlichen kugelsegmentförmig geformt sind. In einigen Ausführungsformen verbessert dies den Fluss von der Mitte des Rotors 130 zur radial äußeren Beschaufelung 138 hinweg. Am stromauf gelegenen Ende der Beschaufelung 138 befindet sich ein Ring 140, welcher zum Ring 116 korrespondierend angeordnet ist. Ferner umfasst der Rotor 130 einen weiteren Ring 142, welcher mit Ring 120 korrespondiert. Sowohl der Ring 140 als auch der Ring 142 sind durch Magnete, insbesondere Permanentmagnete, gebildet, und sind in ihrer Magnetisierung zu den Magnetisierungen der zu ihnen korrespondierenden Ringe entgegengesetzt. Alternativ können die Ringe Weicheisen oder andere magnetisierbare Materialien aufweisen.

In einer weiteren Ausführungsform können die Ringe 116 und 120 im Wesentlichen in axialer Richtung zweigeteilt sein und zwei entgegengesetzt zueinander magnetisierte Ringe umfassen. Auch diese Anordnung der Ringe bewirkt eine passive magnetische Axiallagerung.

In der Blutpumpe 100 der Fig. 2 ist zudem eine Sensorspule 144 angeordnet, welche aufgrund einer durch eine axiale Bewegung des Rotors induzierten Spannung eine Position des Rotors ableiten kann. In einer anderen Ausführungsform ist die Spule 144 eine Steuerspule, welche durch aktive Beaufschlagung mit Strom ein Magnetfeld erzeugt, welches beispielsweise mit den Ringen 140 bzw. 142 interagiert und so eine aktive Positionierung des Rotors im Innenraum zulässt. In einer weiteren Ausführungsform sind sowohl eine Sensor- als auch eine Steuerspule vorhanden. Zudem können zwei Sensorspulen oder auch zwei Steuerspulen vorhanden sein.

In der Fig. 3 ist eine weitere Blutpumpe 200 dargestellt. Die Blutpumpe 200 ist ähnlich aufgebaut wie die Blutpumpe 1. Jedoch verfügt die Blutpumpe 200 über ein Gehäuse 202, welches neben einem Einlass 204, welcher entlang einer Achse 206 verläuft, einen Auslass 208 auf, welcher gegenüber dem Einlass um 90° verdreht ist. Der Auslass 208 umfasst eine Spiralkammer 210, wie sie beispielsweise in den Fign. 3a, 3b, 7a, 7b, 7c und 8 der Anmeldung US 2014 017 1727 dargestellt ist. Die dort gezeigten und beschriebenen Details werden vollumfänglich zum Bestandteil der Offenbarung dieser Anmeldung gemacht. Die Spiralkammer 210 schließt sich an den axialen Gehäuseabschnitt 212 an, in welchem sich der Stator 214 sowie der magnetisierte Ring 216 des Axiallagers 218 befinden. Die Spiralkammer weist dabei eine radiale Aufweitung auf, und verläuft schneckenförmig vom Abschnitt 219 zum Abschnitt 220 zum Auslass 222. Aus der Spiralkammer heraus erstreckt sich in stromauf ein Nabenstumpf 224. Der Rotor 230 umfasst einen Zylinder 232 und eine Beschaufelung 234 sowie einen Ring 236, wie er bereits im Zusammenhang mit den Fign. 1 und 2 beschrieben wurde. Die Beschaufelung 234 erstreckt sich vom stromauf vom Ende 238 bis zum stromab gelegenen Ende 240 des Rotors 230. Der Rotor 230 ist dabei derart im axialen Gehäuseabschnitt 212 angeordnet, dass der Nabenstumpf 224 einen Anschlag bildet. Dabei ist der Rotor derart gegenüber dem Nabenstumpf angeordnet, dass er nicht in die Spiralkammer 210 ragt. Von daher kann beispielsweise auch ein Rotor, wie im Zusammenhang mit der Fig. 1 oder mit der Fig. 2 beschrieben, in der Blutpumpe 200 eingesetzt werden. Der Nabenstumpf 224 kann sich über die gesamte axiale Länge der Spiralkammer 210 erstrecken. In weiteren Ausführungsbeispielen ragt der Rotor in die Spiralkammer hinein, wobei das stromab gelegene Ende der Beschaufelung jedoch nicht in die Spiralkammer hineinragt. Der Auslass 208 befindet sich dann zwischen dem stromauf und dem stromab gelegenen Ende des Rotors 230.

In der Fig. 4 ist ein Querschnitt durch die Pumpe 1 bzw. 100 bzw. 200 im Bereich des axialen Gehäuseabschnitts dargestellt. So zeigt die Fig. 4 in Bezug auf die Blutpumpe 1 das Gehäuse 2 sowie den im Innenraum 16 angeordneten Rotor 20. Zu erkennen ist der Zylinder 24 als auch die Beschaufelung 28 und der die Beschaufelung außen umlaufende Ring 32. Zwischen dem Ring und der Innenwand des Gehäuses 2 befindet sich ein Spalt der Spaltbreite r. Diese Spaltweite ist entscheidend dafür, ob zwischen der Außenfläche des Rings 32 und der Innenfläche des Gehäuses 32 ein hydrodynamisches Lager ausgebildet ist oder nicht. Im Gehäuse 2 ist zudem der magnetisierte Ring 14 vorhanden, welcher im Zusammenwirken mit dem Ring 32 eine axiale, passive Lagerung des Rotors im Gehäuse 2 übernimmt. In der Fig. 4 ist zudem deutlich erkennbar, dass der Radius R des Rotors 20 deutlich größer ist als die Breite b des Rings 32.

Die Beschaufelung 28 umfasst im vorliegenden Beispiel drei Wendeln 50, 52 und 54, deren in radialer Richtung gemessene Höhe deutlich kleiner ist als der Radius R des Rotors. Die Breite B einer einzelnen Wendel ist dabei derart bemessen, dass diese kleiner ist als der Abstand zweier benachbarter Wendeln, beispielsweise der Wendeln 52 und 56. In der Fig. 5 ist der Rotor 20 nochmals in einer dreidimensionalen Perspektive dargestellt. Zu erkennen ist der Zylinder 24 sowie der Ring 32 und die Wendel 52, welche sich entlang der Länge L des Rotors 20 um mindestens 270°, bevorzugt um mindestens 360° helixartig um die Außenfläche des Zylinders 24 dreht. Der in der Fig. 2 dargestellte Rotor wäre dementsprechend mit einem weiteren Ring an seinem stromabwärtigen Ende zu versehen.

Obgleich in den Fign. 1, 2 und 3 bislang die im axialen Gehäuseabschnitt angeordneten magnetisierten Ringe jeweils vollständige Ringe waren, können diese sich auch aus einzelnen Segmenten zusammensetzen. Dabei sind vollständig geschlossene Ringe zwar in einigen Ausführungsbeispielen bevorzugt, es können jedoch auch nur Ringabschnitte, d.h. Winkelsegmente von weniger als 360°, bzw. 270°, 180° oder 90° zum Einsatz kommen.

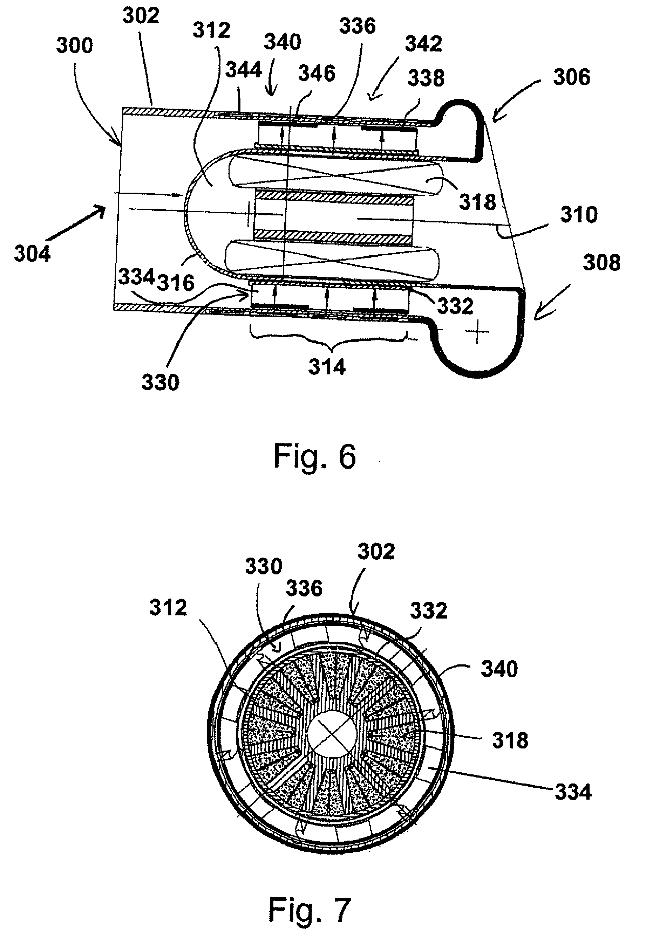

In der Fig. 6 wird eine weitere Ausführungsform einer Blutpumpe dargestellt. Die Blutpumpe 300 umfasst ein Gehäuse 302 mit einem Einlass 304 sowie einem Auslass 306, welcher eine Spiralkammer 308, wie beispielsweise im Zusammenhang mit der Fig. 3 beschrieben, umfasst. Vom auslassseitigen Ende der Pumpe her erstreckt sich entlang der Achse 310 ein Dorn 312, welcher einen zylinderförmigen Abschnitt 314 zwischen dem kugelsegmentförmigen, stromauf gelegenen Ende 316 des Dorns 312 und der Spiralkammer 308 erstreckt. Im Inneren des Dorns befindet sich ein Stator 318, welcher mit Permanentmagneten, welche im Rotor 330 angeordnet sind, interagiert, um den Rotor 330 in Rotation zu versetzen.

Der Rotor 330 umfasst eine Zylinderhülse 332, in welcher beispielsweise Permanentmagnete wie in der US 2014/0171727 beschrieben sind. Alternativ oder zusätzlich hierzu können in der Beschaufelung 334 ebenfalls Permanentmagnete eingesetzt werden. Die Beschaufelung 334 kann beispielsweise wie in den vorherigen Beispielen beschrieben ausgebildet werden. Alternativ kann der Rotor auch derart beschaffen sein, dass die Breite der einzelnen Wendeln breiter ist als ein Abstand zweier benachbarter Wendeln. Am radial äußeren Umfang der Beschaufelung 334 befindet sich ein erster Ring 336 sowie ein stromab davon gelegener zweiter Ring 338. Diese Ringe interagieren mit zwei Ringen 340 bzw. 342, welche im Gehäuse 302 eingelassen sind. Die Ringe 340 bzw. 342 umfassen jeweils zwei Teile (einen ersten Teil 344 und einen zweiten Teil 346), welche in entgegengesetzter Richtung axial magnetisiert sind. Diese Art der Anordnung sichert, dass der Rotor 330 auf dem Dorn gehalten wird. Somit bildet das Zusammenspiel der Ringe 336 bzw. 338 und 340 bzw. 342 eine passive axiale Lagerung 350. Die magnetische zweiteilige Ausführung der Ringe 340 bzw. 342 kann auch bei Pumpen ohne Dorn eingesetzt werden. Weitere für die Pumpe notwendige Komponenten sind in der Darstellung der Fig. 6 nicht eingezeichnet.

In der Fig. 7 ist ein Querschnitt durch die Anordnung der Fig. 6 dargestellt. In der Fig. 7 ist das Gehäuse 302 sowie der Dorn 312 und der Rotor 330 zu erkennen. Das Gehäuse 302 umlaufend ist ein Ring 340 angeordnet. Der Rotor umfasst eine Zylinderhülse 332, eine Beschaufelung 334 sowie einen ersten Ring 336, welcher im Zusammenspiel mit dem Ring 340 eine axiale Lagerung des Rotors 330 auf dem Dorn 312 bewirkt. Eine radiale Lagerung zwischen dem Dorn und dem Rotor wird über den Spalt zwischen der Außenfläche des Dorns 312 und der Innenfläche der Zylinderhülse 332 ausgebildet. Die Spaltbreite ist derart bemessen, dass ein hydrodynamisches Lager gebildet wird, welches den Rotor radial stabilisiert. Ferner ist in der Fig. 6 der Stator 318 zu erkennen, welcher den Rotor antreibt. Weitere Ausführungsbeispiele der hier gezeigten Pumpe mit einer axialen Lagerung mittels eines am äußeren Rand der Beschaufelung angeordneten Rings können beispielsweise der Anmeldung mit dem internen Aktenzeichen 157EP 0367 entnommen werden.

## Patentansprüche

1. Blutpumpe (1; 100; 200; 300), vorzugsweise zur Unterstützung eines Herzens, wobei die Blutpumpe ein Gehäuse (2; 102; 202) mit einem stromauf gelegenen Einlass (4; 104; 204), einem stromab gelegenen Auslass (6; 106; 208; 306) und einen drehbar gelagerten Rotor (20; 130; 230; 330) mit einer Achse und einer Beschaufelung (28; 138; 234; 334) umfasst und der Rotor in axialer Richtung magnetisch gelagert ist,
wobei
mindestens ein, an der Beschaufelung befestigter, die Beschaufelung radial außen umlaufend und in axialer Richtung magnetisierter erster Ring (32; 140, 142; 236; 336, 338) vorhanden ist, und ferner ein, den ersten Ring außen umlaufend, zweiter magnetisierter Ringabschnitt (14; 116; 120; 216; 340, 342) zur Ausbildung eines axialen Magnetlagers (34; 218) in einem ersten axialen Gehäuseabschnitt (8; 110; 212) angeordnet ist und der Rotor eine Zylinderhülse (332) oder einen Zylinder (24; 132; 232) umfasst, wobei die Beschaufelung an einer Außenfläche der Zylinderhülse oder des Zylinders angeordnet ist.

2. Blutpumpe nach Anspruch 1, wobei ein zweiter axialer Teilabschnitt der Beschaufelung einen dritten Ringabschnitt und ein zweiter axialer Gehäuseabschnitt einen vierten, zum dritten Ringabschnitt korrespondierenden Ringabschnitt aufweist.

3. Blutpumpe nach einem der vorhergehenden Ansprüche, wobei mindestens einer der Ringabschnitte einen geschlossenen Ring bildet.

4. Blutpumpe nach einem der vorhergehenden Ansprüche, wobei die Beschaufelung eine Wendel (52, 54, 56) umfasst.

5. Blutpumpe nach Anspruch 4, wobei die Beschaufelung mehrere Wendeln umfasst und zwei benachbarte Wendeln einen Abstand entlang einer Außenfläche des Rotors besitzen, welcher ein Vielfaches einer Breite (B) der Wendeln ist.

6. Blutpumpe nach einem der vorhergehenden Ansprüche, wobei der Rotor in axialer Richtung ausschließlich durch das axiale Magnetlager gelagert ist.

7. Blutpumpe nach einem der vorhergehenden Ansprüche, wobei das axiale Magnetlager derart ausgebildet ist, dass ein stromab gelegenes Ende der Beschaufelung vollständig stromauf des Auslasses angeordnet ist.

8. Blutpumpe nach einem der vorhergehenden Ansprüche, wobei die Beschaufelung derart auf dem Rotor angeordnet ist, dass ein stromab gelegenes Ende des Rotors stromab eines stromab gelegenen Endes der Beschaufelung liegt.

9. Blutpumpe nach einem der vorhergehenden Ansprüche, wobei ein radialer Abstand zwischen einer Innenfläche des Gehäuses und einer Außenfläche des ersten Rings derart ist, dass zwischen der Innenfläche des Gehäuses und der Außenfläche des ersten Rings ein hydrodynamisches Lager ausgebildet ist.

10. Blutpumpe nach einem der Ansprüche 1 bis 9, wobei ein radialer Abstand zwischen einer Innenfläche des Gehäuses und einer Außenfläche des ersten Ringabschnitts derart ist, dass zwischen der Innenfläche des Gehäuses und der Außenfläche des ersten Ringabschnitts kein hydrodynamisches Lager ausgebildet ist.

11. Blutpumpe nach einem der vorhergehenden Ansprüche, wobei der Auslass eine gegenüber der Achse um einen Winkel von mehr als 45°, vorzugsweise zwischen 80° und 100°, verdrehte (geneigte) Auslassrichtung vorgibt.

12. Blutpumpe nach einem der vorhergehenden Ansprüche, wobei zwischen dem Einlass und dem Auslass ein Dorn (312) angeordnet ist, auf welchem der Rotor drehbar gelagert ist.

13. Blutpumpe nach Anspruch 12, wobei zwischen einer Oberfläche des Dorns und einer Innenfläche des Rotors ein hydrodynamisches Lager ausgebildet ist.

14. Blutpumpe nach Anspruch 12 oder 13, wobei ein den Rotor antreibender Stator im Dorn und/oder im ersten Gehäuseabschnitt und/oder proximal des Rotors angeordnet ist.

15. Blutpumpe nach einem der Ansprüche 12 bis 14, wobei der Dorn und ein durch das Gehäuse definierter Innenraum zumindest abschnittsweise koaxial zueinander ausgerichtet sind.

16. Blutpumpe nach einem der vorhergehenden Ansprüche, wobei der erste Ring und/oder der weitere Ringabschnitt Weicheisen aufweist.
